Europäisches Patentamt

European Patent Office

Office européen des brevets

(19)

(11) EP 0 603 040 B1

(12) **FASCICULE DE BREVET EUROPEEN**

(45) Date de publication et mention
de la délivrance du brevet:
**25.03.1998 Bulletin 1998/13**

(51) Int Cl.⁶: **G01N 15/08**, G01N 33/24

(21) Numéro de dépôt: **93402993.5**

(22) Date de dépôt: **13.12.1993**

(54) **Procédé et dispositif perfectionnés pour l'étude des propriétés d'un matériau perméable**

Verbessertes Verfahren und Vorrichtung zur Untersuchung der Eigenschaften eines durchlässigen Materials

Improved method and device for analysing properties of a permeable material

(84) Etats contractants désignés:
**DK FR GB IT NL**

(30) Priorité: **15.12.1992 FR 9215215**

(43) Date de publication de la demande:
**22.06.1994 Bulletin 1994/25**

(73) Titulaire: **INSTITUT FRANCAIS DU PETROLE
92502 Rueil-Malmaison (FR)**

(72) Inventeurs:
• **Fleury, Marc
F-78170 La Celle St Cloud (FR)**

• **Lenormand, Roland
F-92500 Rueil Malmaison (FR)**
• **Ringot, Gabriel
F-92400 Courbevoie (FR)**

(56) Documents cités:
EP-A- 0 206 372      EP-A- 0 473 500
US-A- 4 679 422      US-A- 4 740 077
US-A- 4 868 751      US-A- 4 907 448
US-A- 5 069 065      US-A- 5 079 948

• TRANSACTIONS AIME, vol.160, 1945 pages 114 - 123 G. L. HASSLER ET AL. 'Measurement of Capillary Pressures in Small Core Samples'

## Description

La présente invention a pour objet un procédé et un dispositif perfectionnés pour l'étude des propriétés d'un matériau poreux ou perméable.

Plus particulièrement, l'invention concerne la mesure de la perméabilité relative d'un matériau contenant un fluide en le soumettant successivement à un ou plusieurs cycles comprenant une phase de drainage et à une phase d'imbibition.

Le procédé et le dispositif peuvent trouver des applications dans de nombreux domaines, notamment dans l'étude de gisements pétrolifères. L'évaluation de la richesse et de la productivité d'un gisement souterrain est obtenue par exemple par des simulations numériques à partir de valeurs mesurées de certains paramètres des roches tels que la pression capillaire et leur perméabilité relative pour les fluides qui y sont présents. Ces paramètres sont généralement mesurés en laboratoire à partir d'échantillons de roches prélevées in situ par carottage.

Une méthode de mesure de la pression capillaire consiste par exemple à soumettre des roches poreuses saturées de liquide à une centrifugation à vitesse progressive et à mesurer la quantité de fluide produite en fonction de la vitesse.

On connait une méthode classique pour faire des mesures sur un échantillon saturé successivement dans une phase de drainage et dans une phase d'imbibition. L'échantillon initialement saturé avec un fluide déterminé, est placé dans une enceinte, sur une plaque poreuse laissant passer uniquement le fluide et on injecte un autre fluide sous pression de façon qu'il expulse progressivement le fluide initial. On recueille le fluide expulsé hors de l'échantillon de l'autre côté de la plaque poreuse. Dans une phase d'imbibition, on diminue la pression centrifuge, de façon à étudier la réintégration du fluide initial dans l'échantillon.

Une méthode analogue peut être mise en oeuvre au moyen d'un system où le déplacement des fluides dans un échantillon poreux contenant un fluide (de l'eau par exemple) est obtenu par centrifugation comme décrit dans la demande de brevet FR 2 666 147. Ce système de centrifugation comporte un moteur entraînant en rotation plusieurs bras. Les échantillons sous forme de barreaux cylindriques éventuellement gainés sur leur pourtour, sont placés dans des godets contenant un autre fluide tel que de l'huile par exemple, disposés respectivement à l'extrémité des bras de façon que la force centrifuge chasse le fluide le plus dense radialement hors des échantillons. La progression radiale des fluides à l'intérieur du barreau est déterminée en mesurant les variations du temps de propagation d'ondes acoustiques au travers du barreau au fur et à mesure du déplacement des fluides. Plusieurs couples de transducteurs émetteurs et récepteurs d'ultra-sons sont disposés face à face, en des emplacements différents le long de chaque godet. Les fluides évacués se retrouvent dans une cavité laissée libre dans la partie périphérique du godet.

La demande EP-A-0 206 372 décrit un système de centrifugation semblable, où la distribution des fluides est déterminée au moyen d'un système d'imagerie utilisant des rayons X ou des rayons gamma.

Avec les dispositifs décrits dans les brevets précités, on peut déterminer commodément le phénomène d'expulsion d'un fluide hors d'une roche poreuse au fur et à mesure que la force centrifuge augmente. Mais le phénomène inverse d'imbibition spontanée d'une carotte par le fluide expulsé au préalable qui peut fournir des données complémentaires sur le matériau, ne peut être observé du fait qu'il n'y a généralement plus contact entre l'échantillon et l'interface entre les deux fluides, le plus dense des deux ne pouvant de ce fait le réimbiber durant la phase de décélération.

Le procédé selon l'invention permet d'échapper aux inconvénients ci-dessus mentionnés et d'obtenir des résultats parfaitements reproductibles. Il comporte l'application d'une force centrifuge à un échantillon perméable ou poreux contenant au moins deux fluides de densités différentes, avec une phase de croissance de la force jusqu'à une valeur maximale puis une phase de décroissance, et la mesure en fonction de l'intensité de la force centrifuge appliquée, du déplacement des fluides vers l'extérieur de l'échantillon.

Le procédé est caractérisé en ce qu'il comporte le maintien de l'échantillon en contact avec les deux fluides au moins durant la phase de décroissance de la force centrifuge, de manière à mesurer le déplacement au moins du fluide le plus dense réintégrant l'échantillon en fonction de ladite force centrifuge.

Le procédé comporte par exemple le suivi de l'interface entre les deux fluides à l'extérieur de l'échantillon durant la phase de croissance et de décroissance ou encore sa stabilisation à un niveau de consigne déterminé passant à l'intérieur du volume de l'échantillon un niveau d'une face extérieure de l'échantillon par exemple. Quand l'échantillon se présente sous la forme d'un barreau disposé parallèlement à la direction de la force centrifuge, on peut stabiliser ladite interface à un niveau intermédiaire entre les deux faces opposées, par exemple au niveau d'une face extérieure par où le fluide le plus dense est expulsé par la force centrifuge.

Le procédé comporte par exemple l'introduction d'un échantillon perméable ou poreux contenant au moins les deux fluides de densité différentes, dans un récipient pourvu d'une cavité pour recueillir le fluide expulsé, la mise en rotation du récipient autour d'un axe de rotation, et le maintien dans la cavité de l'interface entre les fluides (à l'extérieur de l'échantillon) sensiblement au contact de celui-ci et au moins au niveau d'une face de l'échantillon la plus éloignée de l'axe de rotation par transfert des fluides entre le récipient et une cavité auxiliaire.

Le maintien de l'interface au niveau de cette face ou au niveau de consigne, comporte par exemple la détermination de ce niveau par télémétrie acoustique, par conductivité électrique ou encore par des mesures de pressions.

L'invention concerne aussi un dispositif de mise en oeuvre qui comporte au moins un récipient ou godet pourvu d'une cavité interne pour un échantillon en contact avec deux fluides de densités différentes, le récipient étant fixé à l'extrémité d'un bras solidaire d'un axe de rotation, des moyens moteurs pour entraîner le bras en rotation et créer une force centrifuge, des moyens pour suivre les déplacements des deux fluides à l'intérieur de l'échantillon.

Le dispositif est caractérisé en ce qu'il comporte une chambre auxiliaire à volume variable communiquant avec le récipient, des moyens pour déterminer la position de l'interface entre le fluide expulsé par la force centrifuge et l'autre fluide (à l'extérieur de l'échantillon) et un ensemble de pilotage pour commander des transferts de fluides entre ledit récipient et ladite chambre auxiliaire de façon à stabiliser ladite interface et éviter toute perte de contact entre les fluides et l'échantillon.

Le dispositif comporte par exemple un deuxième récipient fixé à l'extrémité d'un bras solidaire du même axe de rotation, un piston adapté à coulisser de façon étanche dans le deuxième récipient et délimitant deux cavités de volumes variables, des moyens pour faire communiquer les deux cavités avec le premier récipient et des deuxièmes moyens moteurs commandés par ledit ensemble de pilotage, pour déplacer le piston dans le deuxième récipient, de façon à maintenir l'interface au contact avec l'échantillon.

Suivant un autre mode de réalisation la chambre auxiliaire où coulisse le piston de compensation peut encore être disposée à côté de la cavité contenant l'échantillon dans le même corps de récipient et communiquer avec elle à ses extrémités.

Les moyens pour suivre les déplacements des fluides peuvent comporter deux ensembles d'éléments transducteurs électroacoustiques répartis à l'intérieur du récipient pour déterminer le temps de propagation d'ondes acoustiques au travers dudit échantillon, et que l'on dispose par exemple sur des barrettes de support associées à des moyens de connexion au système de pilotage.

Quand l'un des deux fluides est conducteur de l'électricité, les moyens pour déterminer l'emplacement de l'interface comportent par exemple des éléments conducteurs ayant chacun une extrémité disposée à un niveau différent voisin dudit niveau de consigne ou du niveau de contact, ces éléments conducteurs étant reliés à une source électrique. Ces éléments conducteurs peuvent également être disposés sur une barrette de support associée à des moyens de connexion au système de pilotage.

Les moyens pour déterminer l'emplacement de l'interface (à l'extérieur de l'échantillon) comportent par exemple des éléments de sondage acoustique par écho pour déterminer les variations du niveau de l'interface, ou bien encore des capteurs de pression.

Le dispositif peut comporter aussi deux autres bras disposés de façon à équilibrer l'axe de rotation, et pourvus chacun d'un récipient, l'un d'entre eux comportant des moyens transducteurs électro-acoustiques pour réaliser une compensation en température permettant de corriger des dérives pouvant affecter ledit ensemble de transducteurs du premier récipient.

Le procédé selon l'invention évite toute perte de contact entre les deux fluides et l'échantillon. Il permet une imbibition spontanée de l'échantillon qui a été draîné durant une phase de centrifugation précédente et donc d'observer le déplacement inverse de l'interface entre les deux fluides durant la phase suivante de décroissance jusqu'à son annulation. La connaissance précise de la saturation dans l'échantillon et du niveau de l'interface hors de celui-ci qui est mesurée en permanence, rend possible la détermination de la pression capillaire dans la partie du barreau entre l'interface et la face la plus proche de l'axe de rotation.

Quand on maintient en outre l'interface entre les deux fluides à un niveau de consigne déterminé et notamment au contact de la base du barreau d'échantillon, on peut observer sans rupture de continuité, tout le processus de draînage-imbibition, et déterminer les perméabilités relatives avec une position limite constante.

D'autres caractéristiques et avantages du procédé et du dispositif selon l'invention apparaîtront mieux à la lecture de la description ci-après de modes de réalisation décrits à titre d'exemples non limitatifs, en se référant aux dessins annexés où:

- la Fig.1 montre des courbes représentatives des variations de la saturation d'un échantillon au cours d'un cycle de drainage imbibition;
- la Fig.2, 3 sont des schémas illustratifs de la méthode selon l'invention;
- la Fig.4 montre schématiquement l'agencement d'un tourniquet de centrifugation dans un mode de réalisation où l'on réalise un maintien de l'interface entre les fluides à un niveau de consigne;
- la Fig.5 représente en coupe transversale l'agencement d'un godet contenant un échantillon;
- la Fig.6 montre le même godet en coupe transversale;
- la Fig.7 montre un détail d'une barrette de mesure acoustique;
- la Fig.8 montre un moyen électrique de mesure du niveau du fluide expulsé d'un barreau d'échantillon;
- la Fig.9 représente en coupe transversale l'agencement d'un godet à piston déplaçable;
- la Fig.10 est une vue partielle en coupe d'un godet pourvu d'un moyen électro-acoustique de détection du niveau du fluide expulsé d'un barreau d'échantillon;
- la Fig.11 montre un autre mode de mise en oeuvre avec un tourniquet de centrifugation à quatre bras; et

- la Fig. 12 montre un autre mode de réalisation où les cavités de compensation permettant la stabilisation du niveau du fluide expulsé, sont ménagées dans le godet même qui contient l'échantillon.

Les essais portent par exemple sur un échantillon en forme de barreau allongé découpé dans un matériau poreux à étudier. L'échantillon est saturé avec une certaine quantité d'un premier fluide B de l'eau salée par exemple. On le place dans un récipient que l'on remplit avec un autre fluide A, de l'huile par exemple.

Dans une première phase de drainage, on soumet alors l'ensemble à une force centrifuge dirigée suivant la longueur du récipient de manière à exercer sur lui une force d'expulsion qui tend à faire sortir une partie du premier fluide B. Dans le même temps, du fluide A pénètre à l'intérieur de l'échantillon. Les deux fluides se déplacent à l'intérieur de l'échantillon jusqu'à une position d'équilibre où la force due à la pression capillaire dans les pores, compense la force centrifuge exercée. On sait que la pression capillaire $P_C$ est reliée à la distance r séparant l'interface de l'axe de rotation par la relation :

$$P_C = P_B - P_A = (\rho_B - \rho_A) \, \omega^2 \, (r^2_{max} - r^2)$$

où $\omega$ est la vitesse angulaire de rotation, $r_{max}$ la distance à la base du barreau de l'échantillon à l'axe, $\rho_B$ et $\rho_A$ les masses spécifiques respectives des fluides B et A.

A partir de la mesure précise de la quantité du fluide initial B extraite en fonction de la force centrifuge exercée, et la variation de la saturation moyenne $S_m$ de l'échantillon en fluide B en fonction de la force centrifuge exercée, qui est obtenue par exemple par détection acoustique comme décrit dans le brevet français précité, on peut déduire la pression capillaire dans l'échantillon.

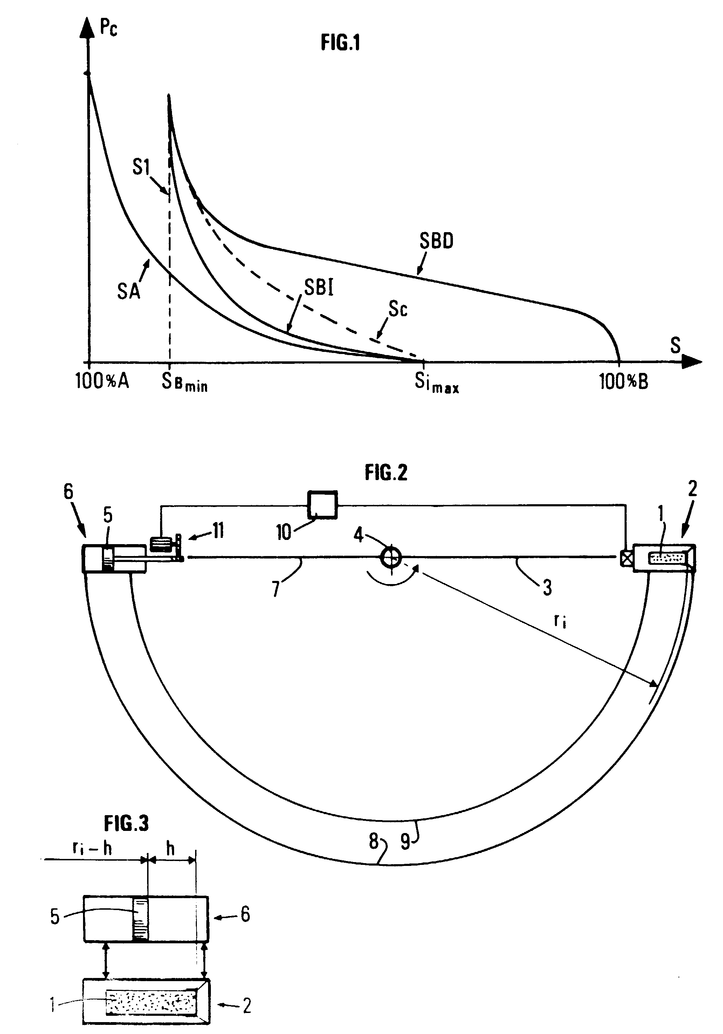

Avec un échantillon saturé d'un fluide B ($S_{Binitiale}$ = 100%), on voit (Fig.1) que la saturation SBD durant la phase de drainage par centrifugation, pour un rayon r déterminé, diminue au fur et à mesure de l'augmentation de la vitesse de rotation $\omega$ jusqu'à une valeur minimale $SB_{min}$. Durant cette phase de drainage, on augmente la vitesse de rotation par paliers successifs jusqu'à atteindre une vitesse de 3500 t/m par exemple.

On procède ensuite à une phase de décélération où la vitesse est ramenée par paliers jusqu'à zéro. Si le fluide B drainé n'est pas au contact du barreau d'échantillon au moment de cette phase de décélaration, la saturation en fluide B reste identique (courbe S1) et l'on perd des données importantes concernant des caractéristiques du matériau poreux.

Le procédé selon l'invention comporte le maintien de l'échantillon au contact de l'interface de deux fluides telle qu'elle existe à l'extérieur de l'échantillon et donc au moins à un niveau minimal. Ce niveau minimal est celui où l'interface effleure la base du barreau c'est-à-dire la plus éloignée de l'axe de rotation ($r_{max}$), et ceci

au moins durant la phase de décélération.

Suivant un premier mode de mise en oeuvre, on suit le déplacement de l'interface entre les deux fluides, extérieurement à l'échantillon, au fur et à mesure que le fluide le plus dense sort de l'échantillon. Il faut dans ce cas que le volume de la chambre à l'intérieur du godet 2 soit assez grand pour recevoir tout le fluide expulsé de l'échantillon.

De préférence, ce niveau d'affleurement est maintenu dès qu'il est atteint lors de de la phase précédente de drainage, de manière à préserver la continuité du processus. Le procédé selon l'invention rend donc possible l'imbibition du matériau poreux et donc la mesure des variations de la saturation en fluide B durant la phase de décélération. On observe un phénomène d'hystérésis et un retour suivant une autre courbe de variation (courbe SBI) jusqu'à un maximum relatif Si max.

On peut procéder ensuite à de nouveaux cycles de drainage-imbibition pour étudier l'évolution des saturations, le premier d'entre eux commençant par une phase de drainage suivant la courbe SC.

Le procédé selon l'invention peut être utilisé aussi pour suivre la saturation progressive par un fluide A d'un barreau d'échantillon initialement saturé complètement en un fluide A à la vitesse de rotation maximale, au fur et à mesure de la décroissance de celle-ci (courbe SA).

Le procédé selon l'invention est mis en oeuvre (Fig. 2) en plaçant un barreau d'échantillon 1 préalablement saturé avec un fluide B tel que de l'eau salée, dans un récipient ou godet 2 contenant un autre fluide A tel que de l'huile. Ce récipient 2 est fixé au bout d'un bras 3 que l'on fait tourner autour d'un axe de rotation 4 à vitesse croissante dans une première phase de drainage, puis décroissante dans une deuxième phase d'imbibition. On détecte quand la position de l'interface entre le fluide B drainé hors du barreau et l'autre fluide A, atteint le niveau de la base du barreau, et si besoin est, l'on maintient ce niveau de consigne en transférant le fluide excédentaire vers une cavité auxiliaire.

Suivant un mode de réalisation, cette cavité auxiliaire est délimitée par un piston 5 mobile dans un autre récipient cylindrique 6 sensiblement de même masse que le premier et comme lui, fixé au bout d'un bras 7 sensiblement de même longueur. Les côtés opposés du piston 5 communiquent par des canalisations souples 8, 9, avec les extrémités opposées du premier récipient.

Un système de régulation 10 connecté à des moyens de détection des déplacements de l'interface, disposés dans le premier godet ou récipient 2, commande si nécessaire des moyens moteurs 11 adaptés à déplacer le piston mobile 5. En situation initiale d'équilibre où aucune parcelle de fluide B ne quitte le barreau, on place le piston 5 à la même distance ri (Fig.2) que la position de consigne entre les deux fluides A et B, de sorte que les pressions de part et d'autre s'équilibrent. A la fin de la phase de draînage où le piston a été déplacé d'une distance h pour assurer le maintien de l'interface en position de consigne, la différence de pression entre les pressions de part et d'autre du piston 5 qui a pour expression:

$$dp = \rho \ \omega^2 \ h \ (ri\text{-}h),$$

dp restant très faible en pratique, de l'ordre de quelques centaines de kPa. Avec cet agencement, des moyens moteurs de puissance relativement faible, suffisent dans la pratique pour déplacer le piston 5.

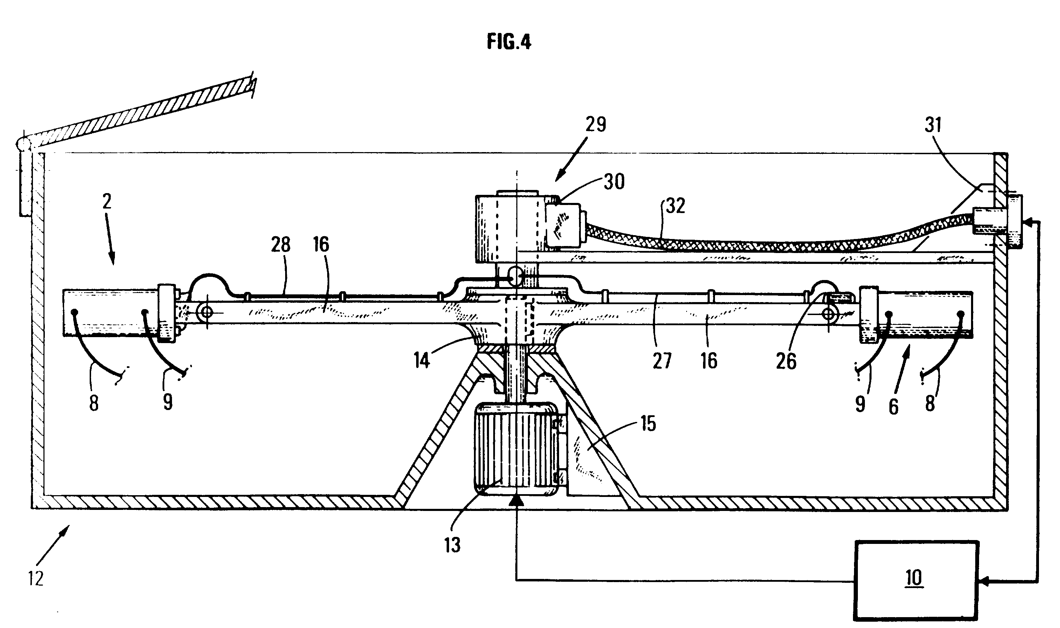

Le mode de réalisation pratique du dispositif de mise en oeuvre de la méthode, illustré aux Fig.4-9, comporte dans une cuve 12, un ensemble de centrifugation de récipients ou godets. Un moteur électrique 13 solidaire de la paroi de la cuve 12, entraîne en rotation un moyeu 14 reposant sur un socle 15, pourvu de deux bras 16 opposés l'un à l'autre et sensiblement de même longueur. Les godets tels que 2, 6, sont montés pivotants aux extrémités respectivement des deux bras 16 de façon à s'aligner spontanément avec la direction de la force centrifuge appliquée.

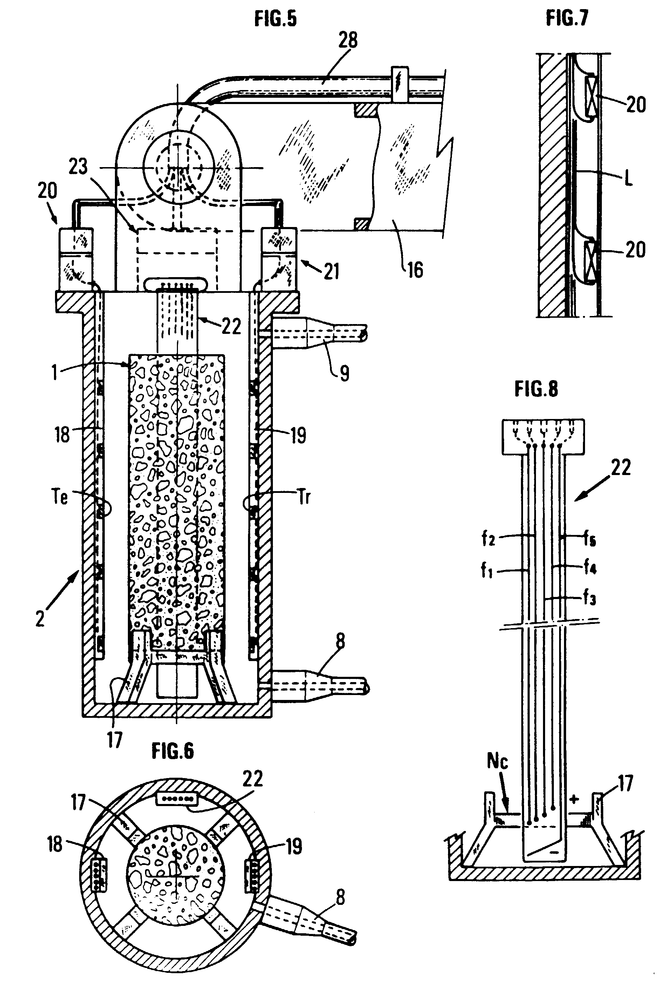

Dans le godet 2 de forme cylindrique par exemple, le barreau à tester est placé sur un support 17. Des moyens acoustiques pour détecter les déplacements des deux fluides à l'intérieur du barreau, sont disposés à plusieurs emplacements définis le long de la cavité intérieure du godet 2, comme décrit dans la demande de brevet FR 2 666 147 déjà citée. Ces moyens comportent deux barrettes de support 18, 19 disposées respectivement dans deux rainures (Fig.6) longitudinales diamétralement opposées de la cavité intérieure. Le long des deux barrettes 18, 19 et à intervalles réguliers, sont fixées respectivement des pastilles piézo-électriques Te, Tr que l'on fait fonctionner, les unes en émetteurs d'ultra-sons, les autres en récepteurs d'ultra-sons.

Les conducteurs électriques L associés à toutes les pastilles de chaque barrette 18, 19 (Fig. 7) sont reliés à une extrémité de celles-ci, respectivement à des connecteurs électriques 20, 21. Chaque barrette avec ses pastilles Te, Tr et ses conducteurs électriques associés, est noyé dans une résine de protection.

En déterminant le temps de propagation d'impulsions entre l'émetteur et le récepteur de chaque couple, qui varie selon que la zone de l'échantillon qu'elles traversent est plus ou moins saturée en fluide A ou B, on peut suivre l'évolution de l'interface au fur et à mesure des variations de la force centrifuge appliquée, comme il est décrit dans la demande de brevet français précitée.

Le dispositif comporte encore des moyens de repérage du niveau de l'interface entre les deux fluides A et B.

Suivant un premier mode de réalisation qui convient quand les deux fluides A et B ont une résistivité électrique très différente l'une de l'autre, les moyens de repérage sont de type électrique. Ils comportent une barrette 22 analogue aux barrettes 18, 19, sur laquelle sont fixées parallèlement plusieurs fils conducteurs de longueurs différentes $P_1$-$P_G$. Il peut s'agir par exemple de pistes réalisées en un métal noble déposées sur une plaquette d'un matériau isolant. Dans l'exemple schématisé à la Fig.8, on utilise quatre pistes f1 à f4 de longueurs inégales dont les extrémités inférieures se répartissent de part et d'autre du niveau de consigne Nc à respecter celui de la base du barreau par exemple. Leur écartement longitudinal est par exemple de quelques dizièmes de millimètre. En opération, une différence de potentiel est établie entre chacune d'elles et la 5ème piste f5. Les cinq pistes sont reliées à une extrémité de la barrette 22, à un connecteur électrique 23.

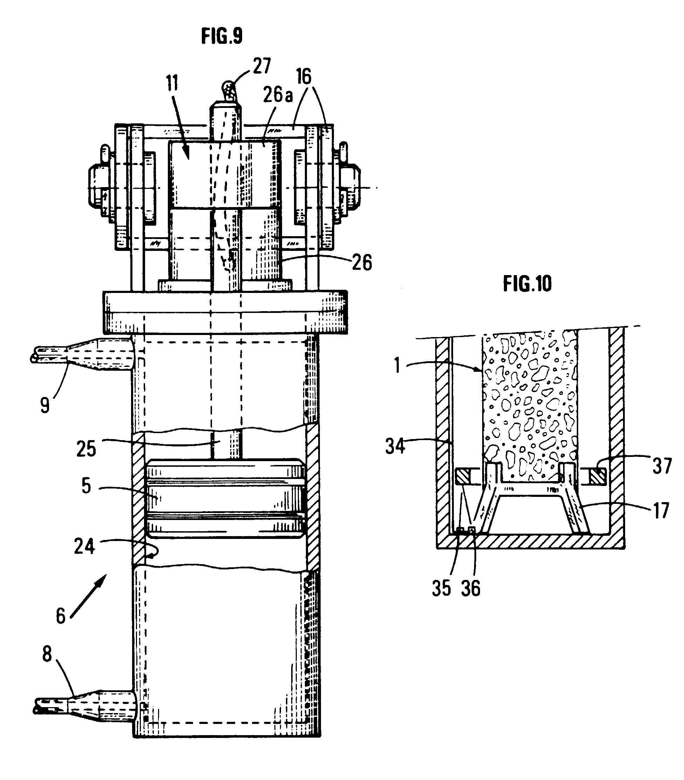

Le godet 6 fixé au bras 16 opposé (Fig.9) comporte une cavité cylindrique 24 où coulisse le piston 5. Les extrémités du godet 6, de part et d'autre du piston 5, communiquent avec les canalisations 8, 9. Au piston 5 est fixée une tige 25 prolongée extérieurement d'une partie filetée. Les moyens moteurs 11 pour déplacer le piston comportent un moteur 26 de type pas-à-pas par exemple, alimenté électriquement par des conducteurs 27 et une boîte de réduction 26A couplée avec la tige filetée 25.

Les différents conducteurs (Fig.5) aboutissant aux connecteurs 20, 21, 23, sont connectés à un faisceau de conducteurs 28. Le faisceau 28 et les conducteurs 27 d'alimentation du moteur 26 (Fig. 4), longent les deux bras 16 jusqu'à un connecteur tournant 29 d'un type connu. La partie périphérique 30 de ce connecteur tournant, est maintenue fixe par une potence 31 fixée à la paroi de la cuve 12, et reliée par un câble collecteur 32 au système de pilotage 10. Celui-ci est adapté:

- à commander le moteur 13 d'entraînement des bras 16 durant les phases de draînage et d'imbition déjà décrites,
- à mesurer la variation de la position de l'interface entre les deux fluides,
- à commander le moteur 26 en fonction des données fournies en permanence par le détecteur électrique 22, de façon à stabiliser s'il y a lieu l'interface entre les deux fluides A, B à un niveau de consigne prédéfini; et
- à commander les cycles de télémétrie acoustique par les transducteurs piézo-électriques des barrettes 18, 19.

Le système de pilotage est par exemple un micro-ordinateur d'un type connu pourvu d'une carte d'interface et programmé pour réaliser la synchronisation des différentes fonctions de commande des moteurs, d'acquisition des données de mesure, de la hauteur de l'interface et s'il y a lieu de régulation à un niveau de consigne.

Suivant un autre mode de réalisation, le moyen de repérage de niveau de l'interface est du type électro-acoustique. Dans ce cas, on remplace la barrette 22, 23 par un barrette 33 encastrable le long de la paroi du godet et pourvue d'une embase 34 pour deux pastilles piézo-électriques 35, 36. La barrette 33 est positionnée pour que l'embase 34 se trouve en opération au-dessous du niveau du fluide expulsé avec les axes des pastilles 35, 36 dirigés vers l'extrémité opposée du godet 2. Dans l'espace périphérique autour du barreau 1, on dispose un flotteur 37 permettant de suivre le niveau de l'interface. Les deux pastilles sont utilisées l'une pour émettre des impulsions acoustiques, l'autre pour recevoir les échos des impulsions émises sur la face inférieure du flotteur 37. La barrette comporte pareillement un passage longitudinal pour les fils conducteurs devant relier les pastilles 35, 36 au système de pilotage 10 via le faisceau 28 (Fig.4) et un connecteur électrique tel que 23. Comme les barrettes 18, 19, la barrette 33 est noyée dans une résine de protection.

Ce mode de réalisation est avantageux du fait que le système de pilotage comporte déjà un ensemble de télémétrie acoustique associé aux pastilles piézo-électriques 20, 21 (Fig.5) et qui peut donc prendre en charge les mesures du niveau de l'interface. Il est également précis car dans la pratique, on arrive à détecter des variations de niveau très inférieures à 1/10eme de mm.

On ne sortirait pas du cadre de l'invention en utilisant un système à capteurs de pression pour mesurer les variations de la position de l'interface entre les deux fluides, au lieu du système de télémétrie acoustique précédent. Un tel système comporte un premier capteur de pression positionné pour être immergé en opération dans le fluide expulsé. Pour compenser les variations de pression mesurées liées directement à la variation de la force centrifuge, on peut placer dans le godet opposé 3 un capteur de pression témoin immergé dans du fluide et combiner les signaux électriques délivrés par les deux capteurs de pression.

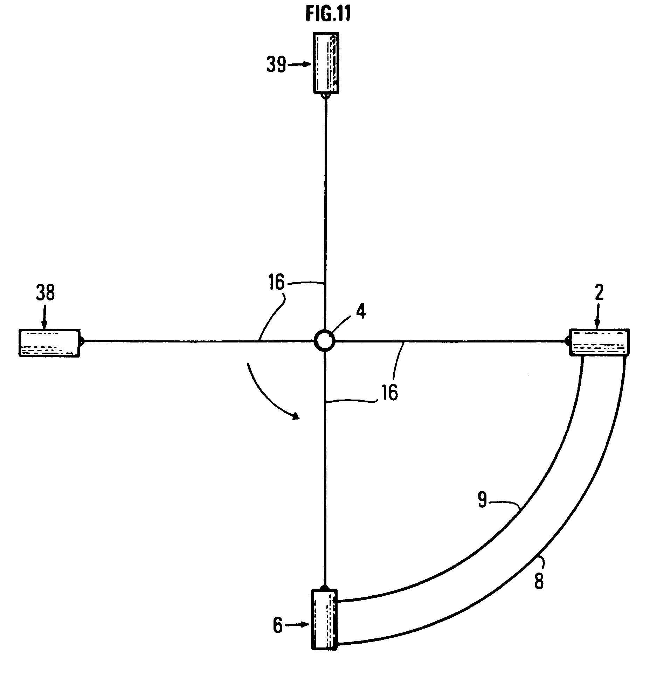

La méthode peut également être mise en oeuvre en utilisant un tourniquet à quatre bras comme schématisé à la Fig.11, qui permet d'obtenir un meilleur équilibrage en rotation et aussi de réaliser des compensations de température. Les deux godets 2 et 6 sont disposés à l'extrémité de deux bras 16 à 90° l'un par rapport à l'autre. Dans un troisième godet 38 analogue au godet 2, on place un barreau d'échantillon de mêmes dimensions que le barreau 1. Ce barreau est saturé de fluide et immergé dans celui-ci. Les mesures télémétriques menées au moyen des barrettes 18, 19 sont utilisées pour compenser en température les mesures analogues obtenues dans le godet 2. Le 4éme godet est utilisé pour équilibrer le godet 3. Il peut comporter également un piston mobile tel que 5 astreint à suivre les déplacements imposés à celui-ci par l'ensemble de pilotage, de façon à obtenir un meilleur équilibrage en rotation.

On a décrit jusqu'ici un dispositif où la cavité de compensation était placée par commodité dans un godet 6 à la même distance de l'axe de centrifugation que le godet contenant le barreau d'échantillon. On ne sortirait pas du cadre de l'invention, cependant, en disposant cette cavité à tout autre endroit, l'essentiel étant le maintien de l'interface entre les deux fluides à un niveau de consigne prédéterminé et de toute façon en contact permanent avec l'échantillon.

Le maintien constant de l'interface au niveau de la base du barreau contribue à simplifier beaucoup la détermination des coefficients de perméabilités relatives qui est effectuée par exemple au moyen de simulations numériques, comme le savent les spécialistes. En maintenant le niveau de l'interface à la base du barreau, on peut considérer que sa longueur qui représente une condition limite imposée pour les calculs est ici constante durant le processus.

Il est possible également de n'utiliser les moyens de mesure de la position de l'interface qu'ils soient acoustiques, électriques ou de pression, que pour obtenir le maintien de l'échantillon en contact avec l'interface entre les deux fluides, et de laisser cet interface se rapprocher librement de l'axe de rotation durant la phase de décélération. Ce mode de mise en oeuvre permet on le rappelle, de déterminer la pression capillaire dans le barreau si l'on connait la position précise de l'interface.

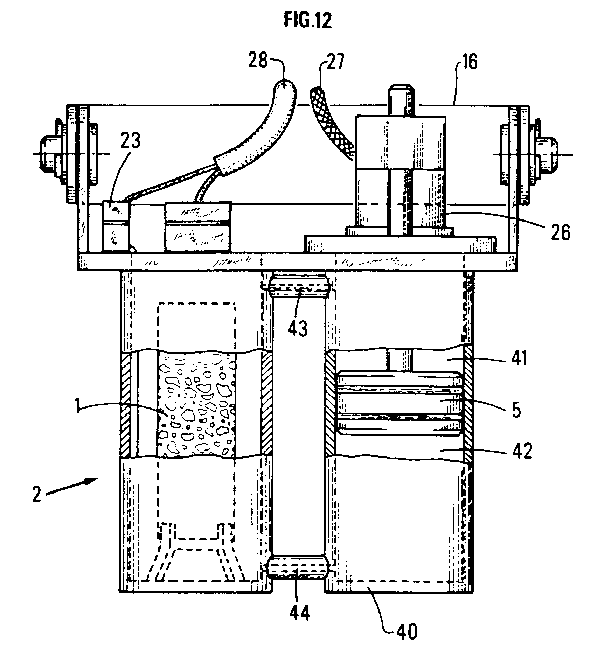

Suivant le mode de réalisation de la Fig. 12 par exemple on place à l'extrémité de l'un des bras 16 du centrifugateur utilisé, un godet 40 dont le corps est pourvu de deux chambres adjacentes 41, 42 dont les parties terminales communiquent deux à deux respectivement par deux canaux 43, 44. Dans la première chambre 41 est placé l'échantillon 1. Dans l'autre 42, coulisse le piston 5 dont le déplacement permet la stabilisation du niveau de l'interface dans la première chambre 41.

A l'extrémité du bras 16 opposé, on place bien évidemment un godet d'équilibrage adapté.

Ce mode de réalisation permet d'éviter les liaisons entre les godets au moyen des câbles 8, 9 précédents.

## Revendications

1. Procédé pour l'étude de propriétés d'un matériau perméable ou poreux par application d'une force centrifuge à un échantillon du matériau (1) en présence d'au moins deux fluides de densité différentes, avec une phase de croisssance de la force centrifuge jusqu'à une valeur maximale puis une phase de décroissance, et la mesure en fonction de l'intensité de la force centrifuge appliquée, du déplacement des fluides vers l'extérieur de l'échantillon, et la détermination suite à cette mesure, de paramètres physiques de l'échantillon, caractérisé en ce qu'il comporte le maintien de l'échantillon en contact permanent avec le fluide le plus dense expulsé, au moins durant toute la phase de décroissance de la force centrifuge où ce fluide expulsé réimbibe l'échantillon.

2. Procédé selon la revendication 1, caractérisé en ce qu'il comporte la stabilisation d'une interface entre les deux fluides extérieurement à l'échantillon à un niveau de consigne Nc permettant le maintien du contact permanent du fluide le plus dense avec l'échantillon.

3. Procédé selon la revendication 2, caractérisé en ce qu'il comporte la stabilisation de ladite interface au niveau d'une face extérieure de l'échantillon.

4. Procédé selon la revendication 2, caractérisé en ce que l'échantillon (1) est un barreau allongé orienté suivant la direction de la force centrifuge appliquée, et en ce qu'il comporte la stabilisation de ladite interface au niveau d'une face de l'échantillon par où le fluide est expulsé sous l'effet de la force centrifuge.

5. Procédé selon la revendication 1, caractérisé en ce qu'il comporte l'introduction d'un échantillon perméable ou poreux contenant au moins les deux fluides de densité différentes, dans un récipient (2) pourvu d'une cavité pour recueillir le fluide expulsé, la mise en rotation du récipient autour d'un axe de rotation, et le maintien de l'échantillon sensiblement au contact des deux fluides et au moins au niveau d'une face de l'échantillon la plus éloignée de l'axe de rotation, par transfert des fluides entre le récipient (2) et une cavité auxiliaire (6).

6. Procédé selon l'une des revendications 2 à 5, caractérise en ce que le maintien de l'interface audit niveau, comporte la détermination de ce niveau par télémétrie acoustique.

7. Procédé selon l'une des revendications 2 à 5, caractérisé en ce que le maintien de l'interface audit niveau, comporte la détermination de ce niveau par conductivité électrique.

8. Procédé selon l'une des revendications 2 à 5, caractérisé en ce que le maintien de l'interface audit niveau comporte la détermination de ce niveau par des mesures de pression.

9. Dispositif pour la mise en oeuvre de la méthode selon l'une des revendications précédentes, comportant au moins un récipient ou godet (2) pourvu d'une cavité interne pour un échantillon (1) en contact avec deux fluides de densités différentes, le récipient étant fixé à l'extrémité d'un bras (16) solidaire d'un axe de rotation, des moyens moteurs (13) pour entraîner le bras en rotation et créer une force centrifuge, des moyens (Te, Tr) pour mesurer les déplacements des deux fluides à l'intérieur de l'échantillon, et des moyens de détermination de paramètres physiques de l'échantillon à partir des mesures de déplacement, caractérisé en ce qu'il comporte une chambre auxiliaire à volume variable (24, 42) communiquant avec le récipient (2), des moyens (22, 34, 35, 36) disposés dans le récipient (2) à l'extérieur de l'échantillon pour déterminer la position de l'interface entre le fluide le plus dense expulsé et l'autre fluide, et un système de pilotage (10) pour commander un transfert du fluide pour déterminer la position de l'interface entre le fluide le plus dense expulsé par la force centrifuge et l'autre fluide le plus dense entre ledit récipient (2) et ladite chambre auxiliaire de façon à stabiliser ladite interface et éviter toute perte de contact entre le fluide le plus dense et l'échantillon.

10. Dispositif selon la revendication 10, caractérisé en ce qu'il comporte un deuxième récipient (6) fixé à l'extrémité d'un bras solidaire du même axe de rotation, un piston (5) adapté à coulisser de façon étanche dans le deuxième récipient et délimitant deux chambres de volumes variables, des moyens (8, 9) pour faire communiquer les deux chambres opposées avec le premier récipient (2) et des deuxièmes moyens moteurs (26) commandés par ledit système de pilotage (10) pour déplacer le piston (5) dans le deuxième récipient (6), de façon à maintenir ladite interface en contact avec l'échantillon.

11. Dispositif selon la revendication 9, caractérisé en ce qu'il comporte un récipient (40) fixé à l'extrémité d'un bras solidaire du même axe de rotation, lequel récipient (40) étant pourvu d'une première chambre (41) pour l'échantillon et d'une deuxième chambre (42) disposée à côté de la première chambre (41) où peut coulisser de façon étanche un piston (5), les deux chambres (41, 42) communiquant l'une avec l'autre à chacune de leurs extrémités par des canaux (43, 44), et des deuxièmes moyens moteurs (26) commandés par ledit système de pilotage (10), pour déplacer le piston (5) dans la deuxième chambre (42) de façon à maintenir ladite interface au contact de l'échantillon.

12. Dispositif selon l'une des revendications 9 ou 11, caratérisé en ce que les moyens pour suivre les déplacements des fluides à l'intérieur de l'échantillon comportent deux ensembles d'éléments transducteurs électro-acoustiques (Te, Tr) répartis à l'intérieur du récipient (2) pour déterminer le temps de propagation d'ondes acoustiques au travers dudit échantillon (1).

13. Dispositif selon la revendication 12, caractérisé en ce que les ensembles de transducteurs (Te, Tr) sont disposés sur des barrettes de support (18, 19) associées à des moyens de connexion (20, 21, 28) au système de pilotage (10).

14. Dispositif selon l'une des revendications 9 à 13, caractérisé en ce que, l'un des deux fluides étant conducteur de l'électricité, lesdits moyens pour déterminer l'emplacement de l'interface comportent des éléments conducteurs (f1-f5) ayant chacun une extrémité disposée à un niveau différent voisin dudit niveau de consigne (Nc), ces élément conducteurs étant reliés à une source électrique.

15. Dispositif selon la revendication 14, caractérisé en ce que les éléments conducteurs sont disposés une barrette (22) de support associée à des moyens de connexion (33) au système de pilotage (10).

16. Dispositif selon l'une des revendications 9 à 15, caractérisé en ce que lesdits moyens pour déterminer l'emplacement de l'interface comportent des éléments (34-37) de sondage acoustique par écho pour déterminer les variations du niveau de l'interface.

17. Dispositif selon l'une des revendications 9 à 15, caractérisé en ce que lesdits moyens pour déterminer l'emplacement de l'interface comportent des moyens de mesure des variations de pression liées à la variation de hauteur du fluide le plus dense dans ladite cavité interne.

18. Dispositif selon l'une des revendications 10 à 15, caractérisé en ce qu'il comporte deux autres bras (16) disposés de façon à équilibrer l'axe de rotation et pourvus chacun d'un récipient, l'un d'entre eux (38) comportant des moyens transducteurs électro-acoustiques connectés au système de pilotage (10) pour réaliser une compensation en température permettant de corriger des dérives de température pouvant affecter ledit ensemble de transducteurs du premier récipient (2).


**Patentansprüche**

1. Verfahren zum Untersuchen der Eigenschaften eines durchlässigen oder porösen Materials durch Ausüben einer Zentrifugalkraft auf eine Probe des Materials (1) in Gegenwart von wenigstens zwei Fluiden unterschiedlicher Dichte, mit einer Phase des Anstiegs der Zentrifugalkraft bis zu einem Maximalwert und dann einer Phase des Abnehmens, und dem Messen, in Abhängigkeit der Stärke der ausgeübten Zentrifugalkraft, der Verschiebung der Fluide zum Äußeren der Probe hin, und nach diesem Messen dem Bestimmen von physikalischen Parametern der Probe, dadurch gekennzeichnet, daß es das Halten der Probe in ständigem Kontakt mit dem ausgetriebenen dichteren Fluid wenigstens während der gesamten Phase des Abnehmens der Zentrifugalkraft umfaßt, wo dieses ausgetriebene Fluid die Probe wieder durchtränkt.

2. Verfahren nach Anspruch 1, dadurch gekennzeichnet, daß es die Stabilisierung einer Grenzschicht zwischen den zwei Fluiden außerhalb der Probe auf einem Sollniveau Nc umfaßt, was es gestattet, den ständigen Kontakt des dichteren Fluids mit der Probe aufrechtzuerhalten.

3. Verfahren nach Anspruch 2, dadurch gekennzeichnet, daß es die Stabilisierung der Grenzschicht in Höhe einer Außenseite der Probe umfaßt.

4. Verfahren nach Anspruch 2, dadurch gekennzeichnet, daß die Probe (1) ein in Richtung der ausgeübten Zentrifugalkraft orientierter länglicher Stab ist und daß es die Stabilisierung der Grenzschicht in Höhe einer Seite der Probe umfaßt, durch die das Fluid unter Wirkung der Zentrifugalkraft ausgetrieben wird.

5. Verfahren nach Anspruch 1, dadurch gekennzeichnet, daß es das Einführen einer durchlässigen oder porösen Probe, die wenigstens die zwei Fluide unterschiedlicher Dichte enthält, in einen Behälter (2), der mit einem Hohlraum zum Aufnehmen des ausgetriebenen Fluids ausgestattet ist, das In-Drehung-Versetzen des Behälters um eine Drehachse und das Halten der Probe im wesentlichen in Kontakt mit den zwei Fluiden wenigstens in Höhe einer von der Drehachse am weitesten entfernten Seite der Probe durch Übertragung der Fluide zwischen dem Behälter (2) und einem Hilfshohlraum (6) umfaßt.

6. Verfahren nach einem der Ansprüche 2 bis 5, dadurch gekennzeichnet, daß das Halten der Grenzschicht auf dem Niveau die Bestimmung dieses Niveaus durch akustische Telemetrie umfaßt.

7. Verfahren nach einem der Ansprüche 2 bis 5, dadurch gekennzeichnet, daß das Halten der Grenzschicht auf dem Niveau die Bestimmung dieses Niveaus durch elektrische Leitfähigkeit umfaßt.

8. Verfahren nach einem der Ansprüche 2 bis 5, dadurch gekennzeichnet, daß das Halten der Grenzschicht auf dem Niveau die Bestimmung dieses Niveaus durch Druckmessungen umfaßt.

9. Vorrichtung zur Durchführung des Verfahrens nach einem der vorhergehenden Ansprüche, mit wenigstens einem Behälter oder Becher (2), der mit einem inneren Hohlraum für eine Probe (1) in Kontakt mit zwei Fluiden unterschiedlicher Dichte ausgestattet ist, wobei der Behälter am Ende eines Arms (16) befestigt ist, der mit einer Drehachse fest verbunden ist, Antriebsmitteln (13) zum Drehantreiben des Arms und Erzeugen einer Zentrifugalkraft, Einrichtungen (Te, Tr) zum Messen der Verschiebungen der zwei Fluide innerhalb der Probe und Mitteln zum Bestimmen physikalischer Parameter der Probe anhand der Verschiebungsmessungen, dadurch gekennzeichnet, daß er eine Hilfskammer mit veränderlichem Volumen (24, 42), die mit dem Behälter (2) kommuniziert, im Behälter (2) außerhalb der Probe angeordnete Mittel (22, 34, 35, 36) zum Bestimmen der Position der Grenzschicht zwischen dem ausgetriebenen dichteren Fluid und dem anderen Fluid und ein Steuersystem (10) zum Steuern einer übertragung von Fluid zum Bestimmen der Position der Grenzschicht zwischen dem durch die Zentrifugalkraft ausgetriebenen dichteren Fluid und dem anderen dichteren Fluid zwischen dem Behälter (2) und der Hilfskammer, um die Grenzschicht zu stabilisieren und jeden Verlust von Kontakt zwischen dem dichteren Fluid und der Probe zu vermeiden.

10. Vorrichtung nach Anspruch 9, dadurch gekennzeichnet, daß sie umfaßt: einen zweiten Behälter (6), der am Ende eines mit derselben Drehachse fest verbundenen Arms befestigt ist, einen Kolben (5), der eingerichtet ist, dicht in dem zweiten Behälter zu gleiten und zwei Kammern mit veränderlichen Volumina begrenzt, Mittel (8, 9) zum In-Kommunikation-Setzen der zwei gegenüberliegenden Kammern mit dem ersten Behälter (2) und zweite Antriebsmittel (26), die von dem Steuersystem (10) gesteuert werden, um den Kolben (5) im zweiten Behälter (6) so zu verschieben, daß die Grenzschicht in Kontakt mit der Probe gehalten wird.

11. Vorrichtung nach Anspruch 9, dadurch gekennzeichnet, daß sie umfaßt: einen am Ende eines mit derselben Drehachse fest verbundenen Arms befestigten Behälter (40), der mit einer ersten Kammer (41) für die Probe und einer neben der ersten Kammer (41) angeordneten zweiten Kammer (42) versehen ist, in der ein Kolben (5) dicht gleiten kann, wobei die zwei Kammern (41, 42) miteinander an jedem ihrer Enden durch Kanäle (43, 44) kommunizieren, sowie zweite Antriebsmittel (26), die von dem Steuersystem (10) gesteuert werden, um die Kolben (5) in der zweiten Kammer (42) so zu verschieben, daß die Grenzschicht in Kontakt mit der Probe gehalten wird.

12. Vorrichtung nach einem der Ansprüche 9 oder 11, dadurch gekennzeichnet, daß die Mittel zum Verfolgen der Verschiebungen der Fluide innerhalb der Probe zwei Anordnungen von elektroakustischen Wandlerelementen (Te, Tr) umfassen, die im Inneren des Behälters (2) verteilt sind, um die Ausbreitungszeit von Schallwellen durch die Probe (1) zu bestimmen.

13. Verfahren nach Anspruch 12, dadurch gekennzeichnet, daß die Anordnungen von Wandlern (Te, Tr) an Trägerstäben (18, 19) angeordnet sind, die Verbindungsmitteln (20, 21, 28) zum Steuersystem (10) zugeordnet sind.

14. Vorrichtung nach einem der Ansprüche 9 bis 13, dadurch gekennzeichnet, daß eines der zwei Fluide elektrisch leitend ist und die Mittel zum Bestimmen der Lage der Grenzschicht leitende Elemente (fl-f5) umfassen, deren jedes ein auf einem anderen Niveau in der Nähe des Sollniveaus (Nc) angeordnetes Ende hat, wobei diese leitenden Elemente an eine elektrische Quelle angeschlossen sind.

15. Vorrichtung nach Anspruch 14, dadurch gekennzeichnet, daß die leitenden Elemente an einem Trägerstab (22) angeordnet sind, der Verbindungsmitteln (33) zum Steuersystem (10) zugeordnet ist.

16. Vorrichtung nach einem der Ansprüche 9 bis 15, dadurch gekennzeichnet, daß die Mittel zum Bestimmen der Lage der Grenzschicht Elemente (34-37) zum akustischen Sondieren durch Echo umfassen, um die Veränderungen des Niveaus der Grenzschicht zu bestimmen.

17. Vorrichtung nach einem der Ansprüche 9 bis 15, dadurch gekennzeichnet, daß die Mittel zum Bestimmen der Lage der Grenzschicht Mittel zum Messen von Druckänderungen umfassen, die mit der Veränderung der Höhe des dichteren Fluids in dem inneren Hohlraum zusammenhängen.

18. Vorrichtung nach einem der Ansprüche 10 bis 15, dadurch gekennzeichnet, daß sie zwei andere Arme (16) umfaßt, die angeordnet sind, um die Drehachse auszuwuchten und deren jeder mit einem Behälter versehen ist, wobei einer von ihnen (38) elektroakustische Wandlermittel umfaßt, die an das Steuersystem (10) angeschlossen sind, um eine Temperaturkompensation durchzuführen, die es gestattet, Temperaturdriften zu korrigieren, die die Anordnung von Wandlern des ersten Behälters (2) beeinflussen können.

**Claims**

1. A method for studying the properties of a permeable or porous material by applying a centrigual force to a sample of material (1) in the presence of at least two fluids of different densities, whereby a phase during which the centrifugal force is increased to a maximum value is followed by a phase of decrease and the displacement of the fluids toward the exterior of the sample is measured as a function of the intensity of the centrifugal force applied, and, after this measurement, physical parameters of the sample are determined, characterised in that it consists in keeping the sample in constant contact with the denser fluid expelled, at least throughout the phase during which the centrifugal force is applied and this expelled fluid is re-imbibed by the sample.

2. A method as claimed in claim 1, characterised in that it consists in stabilising an interface between the two fluids at a reference level Nc externally to the sample which enables the denser fluid to be kept in constant contact with the sample.

3. A method as claimed in claim 2, characterised in that it consists in stabilising the said interface on a level with an external face of the sample.

4. A method as claimed in claim 2, characterised in that the sample (1) is an elongate bar oriented along the direction of the centrifugal force applied and it consists in stabilising the said interface on a level with a face of the sample from which the fluid is expelled under the effect of the centrifugal force.

5. A method as claimed in claim 1, characterised in that t it consists in introducing a permeable or porous sample containing at least the two fluids of different densities into a receptacle (2) provided with a cavity in which the expelled fluid will accumulate, rotating the receptacle about a rotary shaft and maintaining the sample essentially in contact with the two fluids and at least on a level with a face of the sample farthest away from the rotary shaft by transferring fluids between the receptacle (2) and an auxiliary cavity (6).

6. A method as claimed in one of claims 2 to 5, characterised in that the interface is maintained at said level by determining this level using acoustic telemetry.

7. A method as claimed in one of claims 2 to 5, characterised in that the interface is maintained at said level by determining this level using electric conductivity.

8. A method as claimed in one of claims 2 to 5, characterised in that the interface is maintained at said level by determining this level using pressure measurements.

9. A device for implementing the method as claimed in one of the preceding claims, comprising at least one receptacle or bucket (2) provided with an internal cavity for a sample (1) in contact with two fluids of different densities, the receptacle being fixed to the end of an arm (16) integral with a rotating shaft, motor means (13) to drive the arm in rotation and create a centrifugal force, means (Te, Tr) for measuring the displacements of the two fluids inside the sample and means for determining physical parameters of the sample on the basis of the displacement measurements, characterised in that it has an auxiliary chamber of variable volume (24, 42) communicating with the receptacle (2), means (22, 34, 35, 36) arranged inside the receptacle (2) externally to the sample in order to determine the position of the interface between the denser fluid expelled and the other fluid, and a piloting system (10) to control a transfer of fluid to determine the position of the interface between the denser fluid expelled by the centrifugal force and the other denser fluid between the said receptacle (2) and the said auxiliary chamber in order to stabilise said interface and prevent any loss of contact between the denser fluid and the sample.

10. Device as claimed in claim 9, characterised in that it has a second receptacle (6) fixed to the end of an arm integral with the same rotating shaft, a piston (5) designed to slide tightly inside the second receptacle and delineating two chambers of variable volume, means (8, 9) to provide communication between the two opposite chambers and the first receptacle (2) and second motor means (26) controller by the said piloting system (10) so as to move the piston (5) inside the second receptacle (6) in order to maintain said interface in contact with the sample.

11. A device as claimed in claim 9, characterised in that it has a receptacle (40) fixed to the end of an arm integral with the same rotating shaft, said receptacle (40) being provided with a first chamber (41) for the sample and a second chamber (42) arranged alongside the said chamber (41) and in which a piston (5) may slide tightly, the two chambers (41, 42) communicating with one another at each of their ends by means of passages (43, 44), and second motor means (26) controlled by the said piloting system (10) in order to displace the piston (5) in the second chamber (42) so as to maintain the said interface in contact with the sample.

12. A device as claimed in one of claims 9 or 11, characterised in that the means for monitoring the displacements of the fluids inside the sample consist of two sets of electro-acoustic transducer elements (Te, Tr) distributed inside the receptacle (2) in order to determine the time taken to propagate acoustic waves through the said sample (1).

13. A device as claimed in claim 12, characterised in that the sets of transducers (Te, Tr) are arranged on support rods (18, 19) associated with connecting means (20, 21, 28) to the piloting system (10).

14. A device as claimed in one of claims 9 to 13, characterised in that if one of the two fluids is capable of conducting electricity, the said means used to determine the position of the interface consists of conductor elements (f1-f5) each having one end arranged at a different level close to the said reference level (Nc), the conductor elements being connected to a source of electricity.

15. A device as claimed in claim 14, characterised in that the conductor elements are arranged on a support rod (22) associated with means connecting (33) to the piloting system (10).

16. A device as claimed in one of claims 9 to 15, characterised in that the said means used to determine the position of the interface consists of acoustic echo-sounding elements (34-37) for determining changes in the level of the interface.

17. A device as claimed in one of claims 9 to 15, characterised in that the said means used to determine the position of the interface consist of means for measuring variations in pressure linked to the variation in the height of the denser fluid inside the said internal cavity.

18. A device as claimed in one of claims 10 to 15, characterised in that it has two other arms (16) arranged so as to balance the rotating shaft and each provided with a receptacle, one of which (38) has electroacoustic transducer means connected to the piloting system (10) to provide temperature compensation allowing any temperature drifts which might affect the said set of transducers in the first receptacle (2) to be corrected.

**FIG.1**

**FIG.2**

**FIG.3**

FIG.4

**FIG.5**

**FIG.7**

**FIG.8**

**FIG.6**

**FIG.9**

**FIG.10**

**FIG.11**

## FIG.12